# EUROPEAN PATENT SPECIFICATION

(11) **EP 1 259 172 B1**
(45) Date of publication and mention of the grant of the patent: **15.11.2006**
(21) Application number: 00991631.3
(22) Date of filing: 22.12.2000
(51) Int. Cl.: A61B 17/115, A61B 17/11

(54) **SURGICAL STAPLING INSTRUMENT**
CHIRURGISCHES KLAMMERINSTRUMENT
AGRAFEUSE CHIRURGICALE

(30) Priority: 24.01.2000 HU 0000222
(43) Date of publication of application: 27.11.2002
(73) Proprietor: Ethicon Endo-Surgery (Europe) GmbH, 22851 Norderstedt (DE)
(72) Inventor: CSIKY, László, H-9700 Szombathely (HU); NEUROHR, Mark, Newport, KY 41071 (US); BILOTTI, Federico, 00144 Roma (IT); OMAITS, Todd Phillip, Liberty Township, Ohio 45044 (US)
(74) Representative: UEXKÜLL & STOLBERG
(86) International application number: PCT/EP2000/013186
(87) International publication number: WO 2001/054594

(56) References cited:
- US-A- 3 771 526
- US-A- 4 289 133
- US-A- 5 205 459
- US-A- 5 411 508
- US-A- 5 588 579

## Description

The invention relates to a surgical stapling instrument, which can be used for applying surgical staples or clips to tissue and in particular for performing an anastomosis.

Generally, in the performance of a surgical anastomotic stapling operation, two pieces of lumen or tubular tissue, e.g., intestinal tissue, are attached together by a closed row of staples. In performing the anastomosis with a surgical stapling instrument, the two pieces of tubular tissue are clamped together between an anvil provided with an array of staple forming grooves and a staple holder or cartridge device provided with a plurality of staple receiving slots arranged in a closed row or array in which the staples are received. A staple pusher is advanced to drive the staples into the tissue and form the staples against the anvil. Moreover, a circular knife is advanced to cut the excess tissue clamped between the anvil and the staple holder. As a result, the donut-shaped section of tissue is severed from each lumen and remains on the anvil shaft. The tubular tissue joined by the closed row of staples is unclamped by moving the anvil relative to the staple holder, usually by advancing the anvil shaft distally to move the anvil away from the staple holder. The stapling instrument is removed by pulling the anvil through the opening between the pieces of tubular tissue attached by the array of staples.

Surgical stapling instruments of this kind are well-known. For example, U.S. patent No. 5 205 459 describes such an instrument in detail. As usual, the closed row of staples of the instrument disclosed has a planar, circular shape. U.S. patent No. 5 275 322 is a document showing a basic version of a circular stapling instrument.

Although the use of the known surgical stapling instruments is very beneficial and greatly facilitates the performance of an anastomosis, it involves some problems. Often it is difficult to retract the instrument from the site of the operation, because it is difficult to move the anvil through the opening bordered by the closed row of staples, which is somewhat stiff. Moreover, after the operation, the incidence of clinical stenosis at the site of the anastomosis is not rare.

The object of the invention is to provide a surgical stapling instrument for performing an anastomosis, in which said instrument is capable of being removed from the anastomotic site without applying unwanted tissue tension and, in doing so, which reduces the risk of undesirable clinical complications during the healing process.

This object is achieved by a surgical stapling instrument having the features of claim 1. Advantageous versions of the invention follow from the dependent claims.

The surgical stapling instrument according to the invention comprises a frame having a body portion and a handle as well as a staple fastening assembly in the distal region of the instrument. The staple fastening assembly includes a cartridge device, which comprises at least one closed row of staples, and an anvil. The anvil is movable relative to the cartridge device and is adapted to cooperate with the cartridge device for forming the ends of the staples exiting from the cartridge device. A moving device is adapted to move the anvil relative to the cartridge device. A staple driving device is adapted to drive the staples out of the cartridge device towards the anvil. A knife, which has a closed cutting edge, is contained within the cartridge device and is positioned such that there is at least one closed row of staples on the outside of the cutting edge. A knife actuating device is adapted to move the knife towards the anvil. So far, these features are known from the prior art, e.g., from U.S. patent No. 5 205 459.

According to the invention, the line defining the closed row of staples has a wavy shape, leaving (i.e. not being confined to) a plane perpendicular to the longitudinal axis of the staple fastening assembly, such that said line defining the closed row of staples has a larger total length than the projection of said line onto said plane. Preferably, this projection is circular.

The wavy shape of the line defining the closed row of staples can include two complete waves (i.e. two peaks and two valleys), but a number different from two is possible as well.

Thus, the line defining the closed row of staples has a larger total length than the line defining the plane array of staples of a prior art surgical stapling instrument in which the cartridge device has about the same size. Consequently, the length of the anastomosis seam is larger than that of an anastomosis performed by means of a conventional stapling instrument. Because of this increased length, the anastomotic site can assume a larger diameter and is more flexible, so that the anvil can be easily moved through the opening created by the knife, and the surgical stapling instrument can be retracted at the end of the operation more easily. Additionally, the resulting larger anastomosis lumen will alleviate the incidence of clinical complications. On the other hand, if it is sufficient that the total length of the line defining the closed row of staples is comparable to that provided by a conventional stapling instrument, the invention allows the use of a smaller instrument, which generally can be easier inserted into the tubular organ and removed therefrom.

Preferably, the anvil has a wavy-shaped staple-forming surface which matches the wavy shape of the line defining the closed row of staples.

Whereas it is conceivable that the knife has a conventional (cylindrical) basic shape with a circular cutting edge, in a preferred version of the invention the line defining the cutting edge of the knife is wavy-shaped and runs essentially in parallel to the line defining the closed row of staples. In this design, the knife has to be moved by a short distance only in order to cut the tissue clamped in between the cartridge device and the anvil.

In an advantegeous version of the invention, the staple driving device is adapted to drive the staples out of the cartridge device such that each staple is moved essentially along a line which is locally perpendicular to the line defining the closed row of staples. This implies that each of the staples exiting from the cartridge device penetrates the tissue essentially in a perpendicular direction with respect to the local area of the tissue, so that a respective staple passes through the tissue along a relatively short distance. Another advantage is that forces resulting from obliquely driven staples and tending to displace stapled tissue parts with respect to each other do not occur. However, with the wavy shape of the line defining the closed row of staples, generally the staples must be driven and formed at varying angles from the plane perpendicular to the longitudinal axis of the staple fastening assembly. This adds complexity to the staple driving device, and a simple axial drive mechanism, like in conventional stapling instruments, is generally not sufficient.

Preferably, the staple driving device comprises first pushers adapted to be moved essentially parallel to the longitudinal axis of the staple fastening assembly for driving the staples located at the peaks and/or in the valleys of the wavy-shaped line defining the closed row of staples. That implies a generally simple design, but does not work for driving staples which are not located at or close to the peaks and/or the valleys of this wavy-shaped line.

In an advantageous version, the staple driving device also comprises second pushers having a sloped cam surface, each engaging at an intermediate element, which is guided in a slot and adapted to push a staple guided in a slot, wherein the staple driving device is adapted to move the second pushers essentially parallel to the longitudinal axis of the staple fastening assembly for driving staples located in the areas between the peaks and the valleys of the wavy-shaped line defining the closed row of staples via the intermediate elements in a non-parallel manner with respect to said longitudinal axis. This design enables a translation of an axial movement of the second pushers, which can be easily transmitted, e.g. along the body portion or the shaft of the stapling instrument, into an oblique movement required by the staples in question. Moreover, the sloped or wedge-like cam surface of the second pushers has the mechanical advantage that the operator or surgeon requires a reduced force to drive the staples in question through the tissue (i.e. to "fire" these staples). Preferably, each second pusher has two sloped cam surfaces and is adapted to drive two staples towards the anvil, in different directions. A symmetry of the cam surfaces of a second pusher has the benefit of balancing of reaction forces and reducing friction, thus allowing for a more efficient instrument.

Preferably, the first pushers are arranged to drive the staples of a first closed row of staples and the second pushers are arranged to drive the staples of a second closed row of staples, the staples of said first row and of said second row being staggered to each other. Moreover, the staple driving device can be arranged to drive the staples of the first closed row of staples and the staples of the second closed row of staples at different instants. In comparison to conventional stapling instruments, in which all staples are fired simultaneously, a reduced force is required by the surgeon to fire the staples due to the stagger of the peak staple formation load of the first row and the second row of staples.

The first pushers and/or the second pushers can be individually shaped, but they can also be integrally combined in a sleeve structure.

Preferably, the anvil comprises a counterpart adapted to accomodate the cutting edge of the knife. The counterpart preferably matches the wavy shape of the anvil and/or the knife. Such counterparts, which exert a reaction force onto the tissue during the cutting action and are cut by the knife, are generally known in the prior art, see, e.g., US 4 289 133.

In an advantageous version of the invention, the staple fastening assembly is removably mounted in the distal end region of the body portion. This enables, e.g., the staple fastening assembly to be exchanged during a surgical operation or to be designed as a disposable part (whereas the frame including a major part of the mechanical components is sterilizable and re-usable). Moreover, the cartridge device can comprise a removable cartridge containing the staples, such that, e.g., an empty cartridge can be replaced by a fresh one, if required, or the cartridge device can be designed as a re-usable component. Preferably, the anvil is removable as well, which is also essential for certain surgical techniques. To this end, the anvil can comprise a shaft fitting onto a peg protruding from the cartrige device. The peg preferably comprises a mandrel which is useful for piercing tissue in certain surgical techniques. These features are generally known from the prior art surgical stapling instruments.

Generally, the term "staple" is used herein in a very general sense. It includes metal staples or clips, but also surgical fasteners made of synthetic material and similar fasteners. Synthetic fasteners usually have a counterpart (retainer member) held at the anvil. In this sense, the term "anvil" also has a broad meaning which includes, in the case of two-part synthetic fasteners, the anvil-like tool where the retainer members are held, and similar devices.

In the following, the invention is described in more detail by means of embodiments. The drawings show in
- Figure 1: a schematic isometric view of the staple fastening assembly of a first embodiment of the surgical stapling instrument according to the invention, the anvil being removed from the cartridge device (open position),
- Figure 2: isometric representations of the geometry of a (circular) intestine seam made by (A) a conventional surgical stapling instrument having a plane staple line and (B) the surgical stapling instrument of Figure 1 having a wavy-shaped staple line,
- Figure 3: a schematic view of the instrument of Figure 1 when it is inserted into the intestine (rectum) and the anvil is closed, a wavy-shaped intestinal seam being shown,
- Figure 4: a view similar to Figure 3, the intestinal seam being shown in a plane state, when it has a functionally longer perimeter, and the anvil being represented in an open position,
- Figure 5: a side view of a second embodiment of the surgical stapling instrument according to the invention,
- Figure 6: a magnified view of the distal end section of the stapling instrument of Figure 5,
- Figure 7: an isometric view onto the staple-forming face of the anvil of the stapling instrument of Figure 5,
- Figure 8: an isometric view of the circular knife and its counterpart of the stapling instrument of Figure 5,
- Figure 9: a partially sectional view of the counterpart of Figure 8,
- Figure 10: a side view of the distal section of the staple driving device of the stapling instrument of Figure 5,
- Figure 11: a top view of the staple driving device of Figure 10,
- Figure 12: and Figure 13 close-up views of details of the staple driving mechanism of the stapling instrument of Figure 5,
- Figure 14: an isometric view of the end section of the staple driving device of another embodiment of the surgical stapling instrument according to the invention, and
- Figure 15: a side view of the end section of the staple driving device of Figure 14.

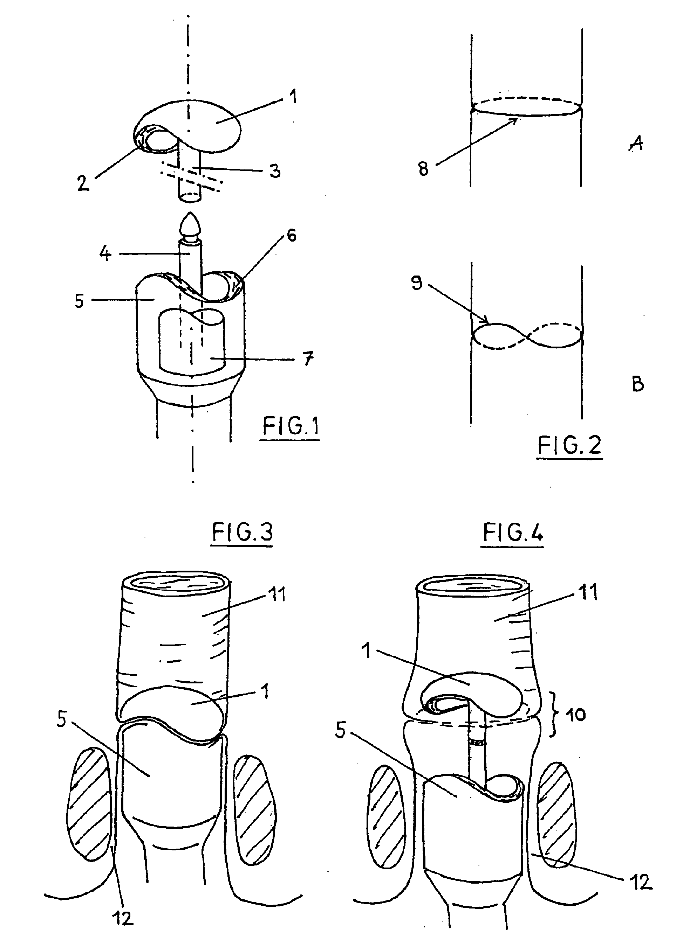

Figures 1 to 4 illustrate the basic concept and the function of the surgical stapling instrument according to the invention by means of an embodiment. Figure 1 shows the distal end section of the stapling instrument.

As can be seen in Figure 1, a wavy-shaped staple-forming face 2 (having a circular cross section) of an anvil 1 and a wavy-shaped end face 6 of a cartridge device 5 (having a circular cross section as well) can be fitted to each other. The connection of the anvil 1 and the cartridge device 5 is ensured by a shaft 3 rigidly fasted to the anvil 1 and a lowerable and raisable peg 4 (which comprises a mandrel) accommodated in the center of the cartridge device 5. A cutter (knife) 7 having a wavy-shaped cutting edge is positioned within the cartridge device 5. In Figure 1, two closed rows of staples, which follow the wavy-shaped end face 6 of the cartridge device 5, are indicated by short dashes.

The stapling instrument of Figure 1 is operated in the following way: In the usual manner, the anvil 1 is inserted into the oral end 11 of the intestine (rectum) of a patient, and the cartridge device 5 is inserted into the aboral end 12 of the intestine (rectum) (Figure 3). Following this, peg 4 is attached to sleeve 3 and then, by lowering peg 4, the wavy-shaped staple-forming face of anvil 1 and the wavy-shaped end face 6 of the cartridge device 5 are moved towards each other. After this, using the staples (clips) situated in the cartridge device 5, a wavy-shaped intestine seam is completed by "firing" the instrument. At the same time, the wavy-shaped cutter 7, which has a circular cross section, cuts out the unnecessary intestine parts. By raising peg 4, the instrument is opened and the anvil can be conveniently pulled through the intestine seam 10, which is able to functionally stretch to a longer perimeter (Figure 4).

Figure 2 schematically illustrates the plane intestine seam 8 made by a conventional circular stapling instrument (part A) and the wavy-shaped intestine seam 9 made by the stapling instrument of Figure 1 (part B). It is evident that only intestine seam 9 allows for a larger-diameter passageway, as shown in Figure 4. This facilitates the removal of the instrument and reduces the risk of developing seam insufficiency and stricture of the intestine seam.

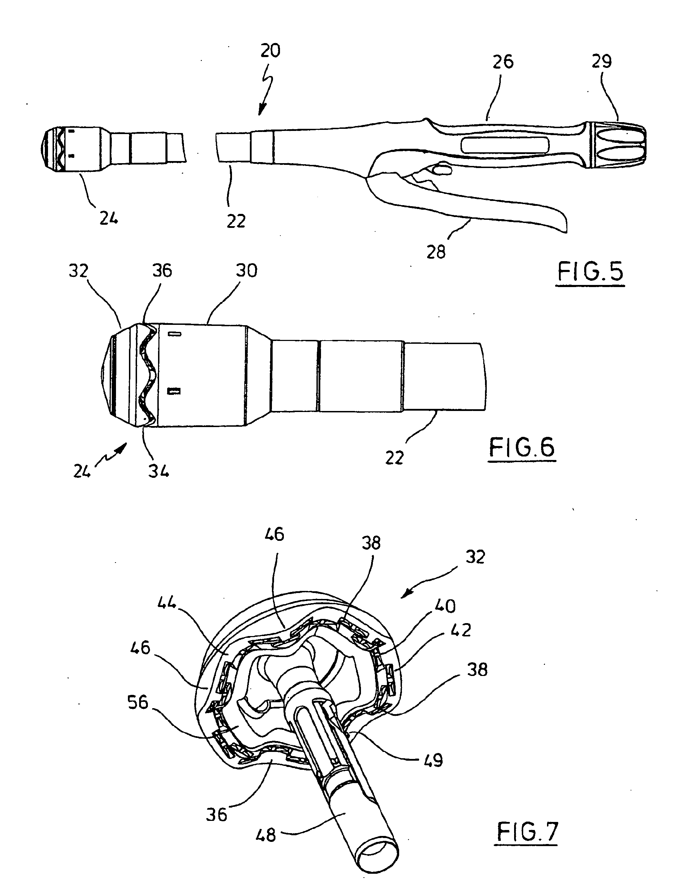

Another embodiment of the surgical stapling instrument according to the invention is explained by means of Figures 5 to 13.

Figure 5 is a side view of the complete surgical stapling instrument 20. The stapling instrument 20 comprises a frame having a body portion including a shaft 22 and a handle 26. A staple fastening assembly 24 is mounted at the distal end of the shaft 22. In the embodiment, the staple fastening assembly 24 can be removed from the shaft 22 and replaced by another one, if desired.

An actuator grip 28 is located in the proximal region of the stapling instrument 20. It can be swivelled towards handle 26 in order to "fire" the stapling instrument 20, i. e. in order to operate the staple driving device and the knife actuating device of the internal mechanism of the stapling instrument. A knob 29 can be rotated in order to axially shift a mandrel comparable to the peg 4 of Figure 1, in order to open or close the staple fastening assembly 24, i. e. in order to move the anvil of the stapling instrument 20.

Generally, the overall design and the mechanical components and drive mechanisms of the stapling instrument 20 are as in conventional circular stapling instruments; the three-dimensional shape of the staple lines and some details of the staple driving device, however, are different, as explained in the following.

Figure 6 is a magnified view of the staple fastening assembly 24 attached to the shaft 22. The staple fastening assembly 24 includes a cartridge device 30 and an anvil 32. The cartridge device 30 comprises a removable cartridge containing two closed rows of staples. The end face 34 of the cartridge device has a wavy shape, as indicated in Figure 6. Thus, the lines defining the closed rows of staples, which essentially follow the end face 34, have a wavy shape as well and are not confined to a plane perpendicular to the longitudinal axis of the staple fastening assembly 24, as in conventional stapling instruments. The anvil 32 has a staple-forming face 36 which is wavy-shaped as well and matches the wavy shape of the end face 34 of the cartridge device 30, see Figure 6.

Figure 7 is an isometric representation of the anvil 32, viewed onto the staple-forming face 36. The staple-forming face 36 comprises a plurality of staple-forming grooves 38, which are arranged in a first closed row 40 on the inner side and a second closed row 42 on the outer side. The staple-forming grooves 38 follow the wavy shape. They also indicate the positions of the corresponding staples in the cartridge device 30, which are arranged in an inner first closed row and an outer second closed row, the individual staples of the rows being staggered to each other. In the embodiment, the wavy shape includes six full wavelengths with peaks at 44 and valleys at 46, see Figure 7. Here, the designations "peak" and "valley" are relative to the wavy shape of the lines defining the closed rows of staples.

The anvil 32 comprises a hollow shaft 48 which can be attached to a peg comprising a mandrel extending along the longitudinal axis of the cartridge device 30, similar to the embodiment shown in Figure 1. A clip mechanism 49 serves to lock the shaft 48 at the mandrel.

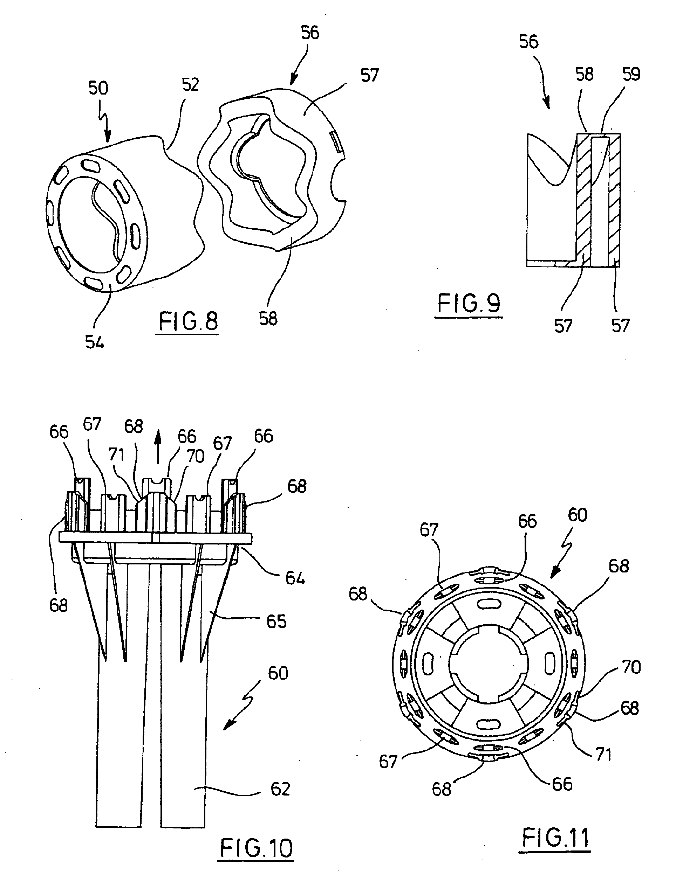

Figure 8 illustrates a knife 50 having a closed and wavy-shaped cutting edge 52. By means of a mounting flange 54, the knife 50 is attached to a knife actuating device such that the knife 50 is contained within the cartridge device 30 on the inside of the inner closed row of staples. The peaks and valleys of the wavy shape of the cutting edge 52 are aligned to the peaks and valleys of the end face 34 of the cartridge device 30. Via the knife actuating device, the knife 50 can be moved towards the anvil 32, when the actuator grip 28 is operated, like in a conventional stapling instrument.

The anvil 32 includes a counter-part 56 which is aligned to the knife 50 when the anvil 32 is attached to the cartridge device 30, see Figure 8 and Figure 7. The counterpart 56 is preferably made of a plastic material and comprises two side walls 57 and, at the wavy-shaped end face 58, a thin wall 59, see Figure 9. During the initial instance of the cutting action, the wall 59 supports the tissue to be cut by the knife 50, and afterwards the cutting edge 52 penetrates the wall 59.

Figure 10 is a side view of the distal section of the staple driving device 60 of the stapling instrument 20. When the actuator grip 28 is operated, the staple driving device 60 is moved in the direction of the arrow shown in Figure 10, which causes the staples to penetrate the tissue clamped in between the cartridge device 30 and the anvil 32. In contrast to conventional staple driving devices, not all of the staples are driven in axial direction, i. e. in the direction of the arrow in Figure 10, because it is intended to move the individual staples in a direction which is essentially perpendicular to the local surface of the end face 34 of the cartridge device 30.

In the staple driving device 60, an actuator shaft 62 guided inside shaft 22 supports a base plate 64 which is reinforced by ribs 65. As shown in Figures 10 and 11, two rows of pushers emerge from the base plate 64, i. e. a circular row of a total of twelve first pushers 66, 67 on the inside and a circular row of a total of six second pushers 68 on the outside. The first pushers include long ones 66 located at the positions of the peaks of the line defining the first closed row of staples and short ones 67 located at the positions of the valleys of this line. All of the second pushers 68 have the same shape and include a sloped cam surface 70 and a sloped cam surface 71, see Figure 10. Each of the second pushers 68 is located close to one of the first pushers 66, see Figure 11.

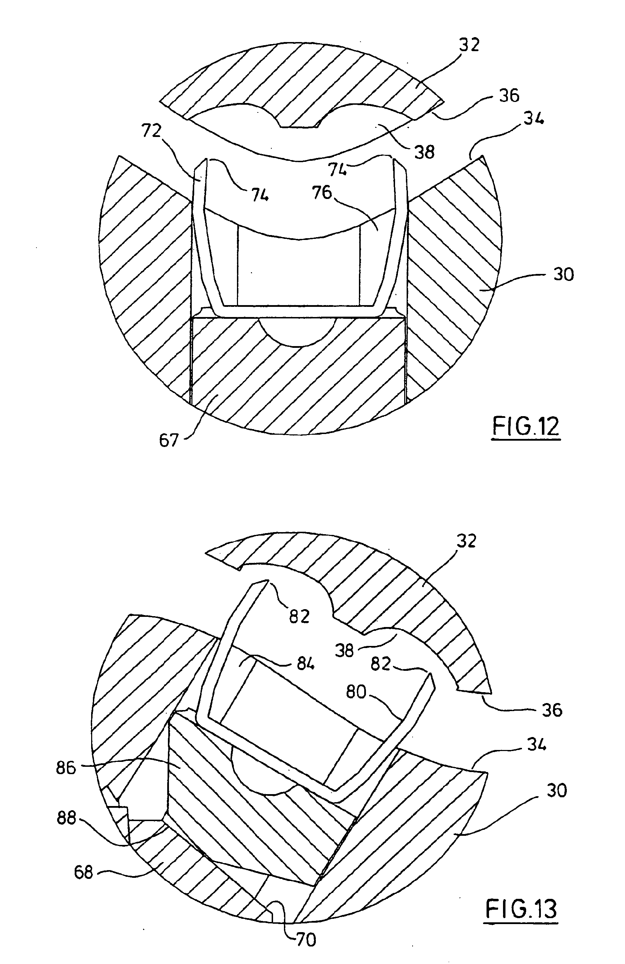

Figure 12 is a magnified longitudinal section which illustrates the operation of one of the first pushers, in this case of a first pusher 67 used to drive a staple 72 located at a valley of the line defining the first closed row of staples. The staple 72 comprises two pointed ends 74 pointing towards the corresponding staple-forming groove 38 at the staple-forming face 36 of anvil 32. Staple 72 is guided inside a slot 76 provided in the material of the cartridge device 30. When the staple driving device 60 is advanced in distal direction, i. e. towards the anvil, the first pusher 67 moves up and drives the staple 72 through the tissue clamped in between the end face 34 of the cartridge device 30 and the anvil 32, until the free ends 74 of the staple 72 are formed by means of the staple-forming groove 38.

A first pusher 66 located at a peak of the staple line works in the same, conventional manner. Since the staples 72 of the first closed row of staples are located at the peaks and valleys of the staple line, a conventional drive mechanism including an axial movement of the first pushers 66, 67 achieves the desired effect that the staples locally penetrate the tissue in a perpendicular manner.

Figure 13 illustrates the operation of a second pusher 68 which is used to drive a staple 80 of the second closed row of staples via the sloped cam surface 70 and at the same time another staple of the second closed row via its other sloped cam surface 71 (see Figure 10). The simultaneous action of the sloped cam surface 71 is not shown in Figure 13, however.

The staple 80 is located in between a peak and a valley of the staple line. In order to achieve a locally perpendicular drive direction, the pointed ends 82 of the staple 80 have to be moved in a non-axial manner towards the corresponding staple-forming groove 38 provided in anvil 32. To this end, the staple 80 is guided in a slot 84 which also guides a flat intermediate element 86. The intermediate element 86 has a bottom side 88 capable of sliding along the sloped cam surface 70. When the second pusher 68 is moved in axial direction, the sloped cam surface 70 pushes the intermediate element 86 which drives the staple 80. In order to allow for a translation of the axial movement of the second pusher 68 into the oblique driving direction of staple 80, the bottom side 88 slides along the sloped cam surface 70.

As already indicated, a second staple 80 is advanced via another intermediate element sliding along the other sloped cam surface 71 of the second pusher 68. Thus, any non-axial forces arising when the staples 80 are driven through tissue largely compensate each other. The sloped cam surfaces 70, 71 have the additional advantage that they act like wedges which reduce the overall force to be exerted by the surgeon when the stapling instrument 20 is operated.

In the embodiment, the lengths of the first pushers 66, 67 and of the second pushers 68 are adjusted such that the staples of the first closed row of staples and the staples of the second closed row of staples are driven through the tissue at different instants, which further reduces the peak load or the maximum force required when the stapling instrument 20 is operated.

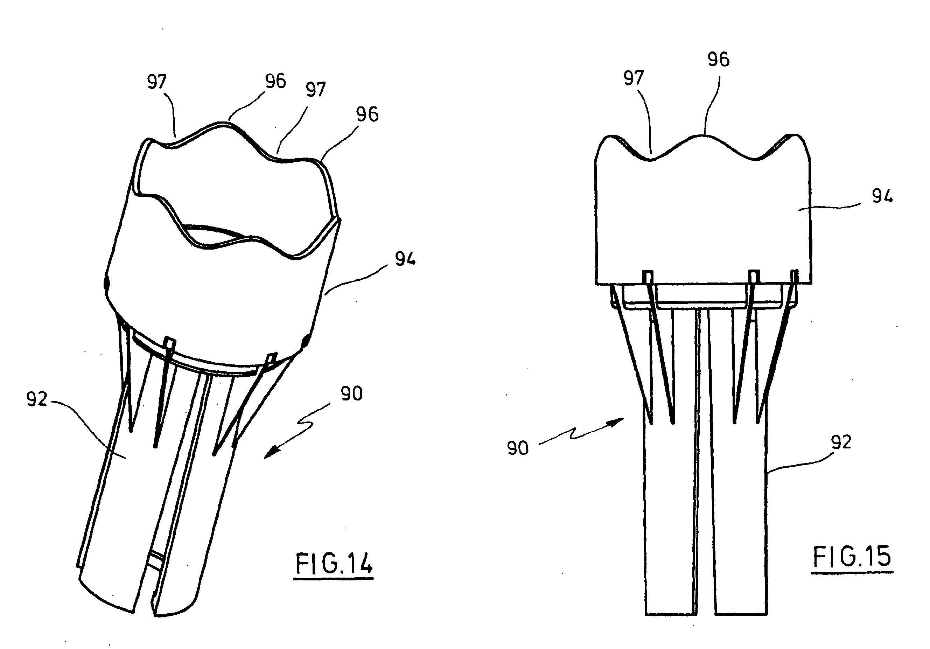

Figures 14 and 15 show another embodiment of the distal section 90 of the staple driving device. In this case, an actuator shaft 92 supports a sleeve structure 94 having a wavy-shaped end with peaks 96 and valleys 97. The sloped areas in between the peaks 96 and the valleys 97 act similar to the sloped cam surfaces 70 and 71 of the embodiment of Figures 10 and 11.

When the surgical stapling instrument 20 is used, its shaft 22 and the staple fastening assembly 24 are introduced into the hollow organ where the anastomosis is to be performed. Generally, the end region of one part of the organ is placed over the end face 34 of the cartridge device 30, whereas the end region of the other part is placed over the anvil 32 such that the anvil shaft 48 protrudes and connects or can serve to connect the anvil 32 to the cartridge device 30. By means of the moving device mentioned above, which is operated via the knob 29, the anvil 32 is moved relative to the cartridge device 30 until the remaining gap between the anvil 32 and the cartridge 30 device is essentially filled by the tissue of both parts of the hollow organ. In a preferred embodiment of the surgical stapling instrument, it is possible to adjust the size of this gap in a well-defined manner. Thereafter, the staple driving device 60 is actuated by means of the actuator grip 28 in order to drive the staples out of the cartridge device 30 towards the anvil 32. The pointed ends of the staples penetrate the tissue in-between and are bent by the anvil 32 so that the staples are closed. Afterwards, the knife actuating device moves the knife 50 towards the anvil 32 so that the cutting edge 52 of the knife 50 penetrates the tissue. The knife actuating device is coupled to the staple driving device so that only actuator grip 28 has to be operated in order to perform the functions of stapling and cutting the tissue almost simultaneously. The presence of two closed rows of staples, which are staggered with respect to each other, ensures a reliable connection of both parts of the hollow organ.

At the end of the procedure, the surgical stapling instrument 20 can be retracted after the gap between the cartridge device 30 and the anvil 32 has been increased in order to release the clamping action. The shape of the closed rows of staples provides a relatively large overall length of the staple lines, so that the tissue at the anastomotic site can yield and the anvil 32 can be easily moved through the opening created by the cutting edge 52 of the knife 50. The excised tissue portion remains in the surgical stapling instrument 20.

## Claims

1. A surgical stapling instrument comprising:
- a frame having a body portion (22) and a handle (26),
- a staple fastening assembly (1, 5; 24) in the distal region of said instrument, the staple fastening assembly (1, 5; 24) including a cartridge device (5; 30), which comprises at least one closed row of staples, and an anvil (1; 32), which is movable relative to the cartridge device (5; 30) and is adapted to cooperate with the cartridge device (5; 30) for forming the ends of the staples exiting from the cartridge device (5; 30),
- a moving device adapted to move the anvil (1; 32) relative to the cartridge device (5; 30),
- a staple driving device (60; 90) adapted to drive the staples out of the cartridge device (5; 30) towards the anvil (1; 32),
- a knife (7; 50), which has a closed cutting edge (52), is contained within the cartridge device (5; 30) and is positioned such that there is at least one closed row of staples on the outside of the cutting edge (52), and
- a knife actuating device adapted to move the knife (7; 50) towards the anvil (1; 32),
- **characterized in that** the line defining the closed row of staples has a wavy shape (6; 34), leaving a plane perpendicular to the longitudinal axis of the staple fastening assembly (1, 5; 24), such that said line defining the closed row of staples has a larger total length than the projection of said line onto said plane.

2. Stapling instrument according to claim 1, **characterized in that** said projection of said line defining the closed row of staples onto said plane perpendicular to the longitudinal axis of the staple fastening assembly (1, 5; 24) is circular.

3. Stapling instrument according to claim 1 or 2, **characterized in that** the anvil (1; 32) has a wavy-shaped staple-forming surface (2; 36) which matches the wavy shape (6; 34) of said line defining the closed row of staples.

4. Stapling instrument according to one of claims 1 to 3, **characterized in that** the line defining the cutting edge (52) of the knife (7; 50) is wavy-shaped und runs essentially in parallel to said line defining the closed row of staples.

5. Stapling instrument according to one of claims 1 to 4, **characterized in that** the staple driving device (60; 90) is adapted to drive the staples out of the cartridge device (30) such that each staple is moved essentially along a line which is locally perpendicular to said line defining the closed row of staples.

6. Stapling instrument according to claim 5, **characterized in that** the staple driving device (60) comprises first pushers (66, 67) adapted to be moved essentially parallel to the longitudinal axis of the staple fastening assembly (24) for driving the staples (72) located at the peaks and/or in the valleys of the wavy-shaped line defining the closed row of staples.

7. Stapling instrument according to claim 5 or 6, **characterized in that** the staple driving device (60) comprises second pushers (68) having a sloped cam surface (70, 71), each engaging at an intermediate element (86), which is guided in a slot (84) and adapted to push a staple (80) guided in a slot (84), wherein the staple driving device (60) is adapted to move the second pushers (68) essentially parallel to the longitudinal axis of the staple fastening assembly (24) for driving staples (80) located in the areas between the peaks and the valleys of the wavy-shaped line defining the closed row of staples via the intermediate elements (86) in a non-parallel manner with respect to said longitudinal axis.

8. Stapling instrument according to claim 7, **characterized in that** each second pusher (68) has two sloped cam surfaces (70, 71) and is adapted to drive two staples (80) towards the anvil (32), in different directions.

9. Stapling instrument according to claim 6 and to claim 7 or 8, **characterized in that** the first pushers (66, 67) are arranged to drive the staples of a first closed row of staples and the second pushers (68) are arranged to drive the staples of a second closed row of staples, the staples of said first row and of said second row being staggered to each other.

10. Stapling instrument according to claim 9, **characterized in that** the staple driving device (60) is arranged to drive the staples of the first closed row of staples and the staples of the second closed row of staples at different instants.

11. Stapling instrument according to one of claims 6 to 10, **characterized in that** the first pushers and/or the second pushers are integrally combined in a sleeve structure (94).

12. Stapling instrument according to one of claims 1 to 11, **characterized in that** the anvil (32) comprises a counterpart (56) adapted to accomodate the cutting edge (52) of the knife (50).

13. Stapling instrument according to one of claims 1 to 12, **characterized in that** the staple fastening assembly (24) is removably mounted in the distal end region of the body portion (22).

14. Stapling instrument according to one of claims 1 to 13, **characterized in that** the anvil (1; 32) is removable.

15. Stapling instrument according to claim 14, **characterized in that** the anvil (1; 32) comprises a shaft (3; 48) fitting onto a peg (4) protruding from the cartrige device (5; 30), which peg (4) preferably comprises a mandrel.

16. Stapling instrument according to one of claims 1 to 15, **characterized in that** the cartridge device (5; 30) comprises a removable cartridge containing the staples.

## Patentansprüche

1. Chirurgisches Klammerinstrument mit:
- einem Rahmen mit einem Körperbereich (22) und einem Griff (26),
- einer Klammerbefestigungsanordnung (1, 5; 24) im distalen Bereich des Klammerinstruments, wobei die Klammerbefestigungsanordnung (1, 5; 24) eine wenigstens eine geschlossene Klammerreihe aufweisende Magazineinrichtung (5; 30) und ein Gegenlager (1; 32) aufweist, das relativ zu der Magazineinrichtung (5; 30) bewegbar ist und dazu eingerichtet ist, zum Formen der Enden der aus der Magazineinrichtung (5; 30) austretenden Klammern mit der Magazineinrichtung (5; 30) zusammenzuwirken ,
- einer Bewegungseinrichtung, die dazu eingerichtet ist, das Gegenlager (1; 32) relativ zu der Magazineinrichtung (5; 30) zu bewegen,
- eine Klammertreibereinrichtung (60; 90), die dazu eingerichtet ist, die Klammern aus der Magazineinrichtung (5; 30) auf das Gegenlager (1; 32) zu auszutreiben,
- einem Messer (7; 50), das eine geschlossene Schneidkante (52) hat, in der Magazineinrichtung (5; 30) enthalten ist und derart angeordnet ist, dass sich wenigstens eine geschlossene Klammerreihe außerhalb der Schneidkante (52) befindet, und
- einer Messerbetätigungseinrichtung, die dazu eingerichtet ist, das Messer (7; 50) auf das Gegenlager (1; 32) zu zu bewegen,
- **dadurch gekennzeichnet, dass** die die geschlossene Klammerreihe definierende Linie eine wellenartige Form (6; 34) hat, wobei sie eine Ebene senkrecht zu der Längsachse der Klammerbefestigunganordnung (1, 5; 24) verlässt, so dass die die geschlossene Klammerreihe definierende Linie eine größere Gesamtlänge hat als die Projektion dieser Linie auf diese Ebene.

2. Klammerinstrument nach Anspruch 1, **dadurch gekennzeichnet, dass** die Projektion der die geschlossene Klammerreihe definierende Linie auf die Ebene senkrecht zu der Längsachse der Klammerbefestigungsanordnung (1, 5; 24) kreisförmig ist.

3. Klammerinstrument nach Anspruch 1 oder 2, **dadurch gekennzeichnet, dass** das Gegenlager (1; 32) eine wellenförmige Klammerformungsfläche (2; 36) hat, die zu der wellenartigen Form (6; 34) der die geschlossene Klammerreihe definierenden Linie passt.

4. Klammerinstrument nach einem der Ansprüche 1 bis 3, **dadurch gekennzeichnet, dass** die die Schneidkante (52) des Messers (7; 50) definierende Linie wellenförmig ist und im Wesentlichen parallel zu der die geschlossene Klammerreihe definierenden Linie verläuft.

5. Klammerinstrument nach einem der Ansprüche 1 bis 4, **dadurch gekennzeichnet, dass** die Klammertreibereinrichtung (60, 90) dazu eingerichtet ist, die Klammern aus der Magazineinrichtung (30) so auszutreiben, dass jede Klammer im Wesentlichen entlang einer Linie bewegt wird, die lokal senkrecht zu der die geschlossene Klammerreihe definierenden Linie steht.

6. Klammerinstrument nach Anspruch 5, **dadurch gekennzeichnet, dass** die Klammertreibereinrichtung (60) erste Schiebeelemente (66, 67) aufweist, die dazu eingerichtet ist, im Wesentlichen parallel zur Längsachse der Klammerbefestigungsanordnung (24) bewegt zu werden, um die Klammern (72) zu treiben, die sich an den Gipfeln und/oder in den Tälern der die geschlossene Klammerreihe definierenden wellenförmigen Linie befinden.

7. Klammerinstrument nach Anspruch 5 oder 6, **dadurch gekennzeichnet, dass** die Klammertreibereinrichtung (60) zweite Schiebeelemente (68) mit einer geneigten Kurvenfläche (70, 71) aufweist, die jeweils an einem Zwischenelement (86) angreifen, das in einem Schlitz (84) geführt ist und dazu eingerichtet ist, eine in einem Schlitz (84) geführte Klammer (80) vorzuschieben, wobei die Klammertreibereinrichtung (60) dazu eingerichtet ist, die zweiten Schiebeelemente (68) im Wesentlichen parallel zur Längsachse der Klammerbefestigungsanordnung (24) zu bewegen, um in den Bereichen zwischen den Bergen und den Tälern der die geschlossene Klammerreihe definierenden wellenförmigen Linie befindliche Klammern (80) über die Zwischenelemente (86) in einer nicht parallelen Weise in Bezug auf die Längsachse zu treiben.

8. Klammerinstrument nach Anspruch 7, **dadurch gekennzeichnet, dass** jedes zweite Schiebeelement (68) zwei geneigte Kurvenflächen (70, 71) hat und dazu eingerichtet ist, zwei Klammern (80) in verschiedenen Richtungen auf das Gegenlager (32) zu zu treiben.

9. Klammerinstrument nach Anspruch 6 und nach Anspruch 7 oder 8, **dadurch gekennzeichnet, dass** die ersten Schiebeelemente (66, 67) so angeordnet sind, dass sie die Klammern einer ersten geschlossenen Klammerreihe treiben, und die zweiten Schiebeelemente (68) so angeordnet sind, dass sie die Klammern einer zweiten geschlossenen Klammerreihe treiben, wobei die Klammern der ersten Reihe und der zweiten Reihe gegeneinander versetzt sind.

10. Klammerinstrument nach Anspruch 9, **dadurch gekennzeichnet, dass** die Klammertreibereinrichtung (60) dazu eingerichtet ist, die Klammern der ersten geschlossenen Klammerreihe und die Klammern der zweiten geschlossenen Klammerreihe zu verschiedenen Zeitpunkten zu treiben.

11. Klammerinstrument nach einem der Ansprüche 6 bis 10, **dadurch gekennzeichnet, dass** die ersten Schiebeelemente und/oder die zweiten Schiebeelemente integral in einer Hülsenstruktur (94) kombiniert sind.

12. Klammerinstrument nach einem der Ansprüche 1 bis 11, **dadurch gekennzeichnet, dass** das Gegenlager (32) ein Gegenstück (56) aufweist, das dazu eingerichtet ist, die Schneidkante (52) des Messers (50) aufzunehmen.

13. Klammerinstrument nach einem der Ansprüche 1 bis 12, **dadurch gekennzeichnet, dass** die Klammerbefestigungsanordnung (24) abnehmbar im distalen Endbereich des Körperbereichs (22) montiert ist.

14. Klammerinstrument nach einem der Ansprüche 1 bis 13, **dadurch gekennzeichnet, dass** das Gegenlager (1; 32) abnehmbar ist.

15. Klammerinstrument nach Anspruch 14, **dadurch gekennzeichnet, dass** das Gegenlager (1; 32) einen Schaft (3; 48) aufweist, der auf einen von der Magazineinrichtung (5; 30) vorspringenden Stift (4) passt, wobei der Stift (4) vorzugsweise einen Dorn aufweist.

16. Klammerinstrument nach einem der Ansprüche 1 bis 15, **dadurch gekennzeichnet, dass** die Magazineinrichtung (5; 30) ein entfernbares Magazin aufweist, das die Klammern enthält.

## Revendications

1. Instrument d'agrafage chirurgical comprenant :
- un cadre ayant une partie de corps (22) et une poignée (26),
- un assemblage de fixation d'agrafe (1, 5 ; 24) dans la région distale dudit instrument, l'assemblage de fixation d'agrafe (1, 5 ; 24) incluant un dispositif à cartouche (5 ; 30), qui comprend au moins une rangée fermée d'agrafes, et une enclume (1 ; 32), qui est mobile par rapport au dispositif à cartouche (5 ; 30) et qui est adaptée pour coopérer avec le dispositif à cartouche (5 ; 30) pour former les extrémités des agrafes sortant du dispositif à cartouche (5 ; 30),
- un dispositif mobile adapté pour déplacer l'enclume (1 ; 32) par rapport au dispositif à cartouche (5 ; 30),
- un dispositif d'entraînement d'agrafe (60 ; 90) adapté pour entraîner les agrafes hors du dispositif à cartouche (5 ; 30) vers l'enclume (1 ; 32),
- un couteau (7 ; 50), qui a un bord coupant fermé (52), est contenu dans le dispositif à cartouche (5 ; 30) et est positionné de telle sorte qu'il y a au moins une rangée fermée d'agrafes sur l'extérieur du bord coupant (52), et
- un dispositif d'actionnement du couteau adapté pour déplacer le couteau (7 ; 50) vers l'enclume (1 ; 32),
- **caractérisé en ce que** la ligne définissant la rangée fermée d'agrafes a une forme ondulée (6 ; 34), laissant un plan perpendiculaire à l'axe longitudinal de l'assemblage de fixation d'agrafe (1, 5 ; 24) de telle sorte que ladite ligne définissant la rangée fermée d'agrafes a une longueur totale plus importante que la projection de ladite ligne sur ledit plan.

2. Instrument d'agrafage selon la revendication 1, **caractérisé en ce que** ladite projection de ladite ligne définissant la rangée fermée d'agrafes sur ledit plan perpendiculaire à l'axe longitudinal de l'assemblage de fixation d'agrafe (1, 5 ; 24) est circulaire.

3. Instrument d'agrafage selon la revendication 1 ou 2, **caractérisé en ce que** l'enclume (1 ; 32) a une surface de façonnage d'agrafe de forme ondulée (2 ; 36) qui correspond à la forme ondulée (6 ; 34) de ladite ligne définissant la rangée fermée d'agrafes.

4. Instrument d'agrafage selon l'une des revendications 1 à 3, **caractérisé en ce que** la ligne définissant le bord coupant (52) du couteau (7 ; 50) est de forme ondulée et court essentiellement parallèle à ladite ligne définissant la rangée fermée d'agrafes.

5. Instrument d'agrafage selon l'une des revendications 1 à 4, **caractérisé en ce que** le dispositif d'entraînement d'agrafe (60 ; 90) est adapté pour entraîner les agrafes hors du dispositif à cartouche (30) de telle sorte que chaque agrafe est déplacée essentiellement le long d'une ligne qui est localement perpendiculaire à ladite ligne définissant la rangée fermée d'agrafes.

6. Instrument d'agrafage selon la revendication 5, **caractérisé en ce que** le dispositif d'entraînement d'agrafe (60) comprend des premiers poussoirs (66, 67) adaptés pour être déplacés de façon essentiellement parallèle à l'axe longitudinal de l'assemblage de fixation d'agrafe (24) pour entraîner les agrafes (72) situées sur les sommets et/ou dans les vallées de la ligne de forme ondulée définissant la rangée fermée d'agrafes.

7. Instrument d'agrafage selon la revendication 5 ou 6, **caractérisé en ce que** le dispositif d'entraînement d'agrafe (60) comprend des seconds poussoirs (68) ayant une surface de came pentue (70, 71), chacun étant en prise à un élément intermédiaire (86), qui est guidé dans une fente (84) et adapté pour pousser une agrafe (80) guidée dans une fente (84), dans lequel le dispositif d'entraînement d'agrafe (60) est adapté pour déplacer les seconds poussoirs (68) de façon essentiellement parallèle à l'axe longitudinal de l'assemblage de fixation d'agrafe (24) pour entraîner les agrafes (80) situées dans les parties entre les sommets et les vallées de la ligne de forme ondulée définissant la rangée fermée d'agrafes via les éléments intermédiaires (86) selon une manière qui n'est pas parallèle par rapport au dit axe longitudinal.

8. Instrument d'agrafage selon la revendication 7, **caractérisé en ce que** chaque second poussoir (68) a deux surfaces de came pentues (70, 71) et est adapté pour entraîner deux agrafes (80) vers l'enclume (32), dans des directions différentes.

9. Instrument d'agrafage selon la revendication 6 et la revendication 7 ou 8, **caractérisé en ce que** les premiers poussoirs (66, 67) sont arrangés pour entraîner les agrafes d'une première rangée fermée d'agrafes et les seconds poussoirs (68) sont arrangés pour entraîner les agrafes d'une seconde rangée fermée d'agrafes, les agrafes de ladite première rangée et de ladite seconde rangée étant échelonnées les unes par rapport aux autres.

10. Instrument d'agrafage selon la revendication 9, **caractérisé en ce que** le dispositif d'entraînement d'agrafe (60) est arrangé pour entraîner les agrafes de la première rangée fermée d'agrafes et les agrafes de la seconde rangée fermée d'agrafes à des instants différents.

11. Instrument d'agrafage selon l'une des revendications 6 à 10, **caractérisé en ce que** les premiers poussoirs et/ou les seconds poussoirs sont intégralement combinés dans une structure à manchon (94).

12. Instrument d'agrafage selon l'une des revendications 1 à 11, **caractérisé en ce que** l'enclume (32) comprend une pièce (56), qui va de pair, adaptée pour loger le bord coupant (52) du couteau (50).

13. Instrument d'agrafage selon l'une des revendications 1 à 12, **caractérisé en ce que** l'assemblage de fixation d'agrafe (24) est monté de manière amovible dans la région d'extrémité distale de la partie de corps (22).

14. Instrument d'agrafage selon l'une des revendications 1 à 13, **caractérisé en ce que** l'enclume (1 ; 32) est amovible.

15. Instrument d'agrafage selon la revendication 14, **caractérisé en ce que** l'enclume (1 ; 32) comprend un arbre (3 ; 48) se fixant sur une cheville (4) faisant saillie du dispositif à cartouche (5 ; 30), laquelle cheville (4) comprend, de préférence, un mandrin.

16. Instrument d'agrafage selon l'une des revendications 1 à 15, **caractérisé en ce que** le dispositif à cartouche (5 ; 30) comprend une cartouche amovible contenant les agrafes.
